# EUROPEAN PATENT SPECIFICATION

(11) **EP 2 498 830 B1**
(45) Date of publication and mention of the grant of the patent: **21.09.2016**
(21) Application number: 10781753.8
(22) Date of filing: 11.11.2010
(51) Int. Cl.: A61L 27/20, A61L 27/52, A61K 47/48

(54) **Dextran-hyaluronic acid based hydrogels**
Hydrogels auf Basis von Dextran-Hyaluronsäure
Hydrogels à base de dextrane-acide hyaluronique

(30) Priority: 11.11.2009 EP 09175724
(43) Date of publication of application: 19.09.2012
(73) Proprietor: University of Twente, Institute for Biomedical Technology and Technical Medicine (MIRA), 7522 NB Enschede (NL)
(72) Inventor: KARPERIEN, Hermanus, Bernardus, Johannes, NL-7151 DJ Eibergen (NL); JIN, Rong, NL-7522 MJ Enschede (NL); MOREIRA TEIXEIRA, Liliana, Sofia, NL-7523 RG Enschede (NL); FEIJEN, Jan, NL-7552 GD Hengelo (NL); DIJKSTRA, Pieter, Jelle, NL-7623 CP Borne (NL)
(74) Representative: V.O.
(86) International application number: PCT/NL2010/050751
(87) International publication number: WO 2011/059325

(56) References cited:
- M. KURISAWA ET AL.: "Injectable biodegradable hydrogels composed of hyaluronic acid-tyramine conjugates for drug delivery and tissue engineering", CHEMICAL COMMUNICATIONS, 28 July 2005 (2005-07-28), pages 4312-4314, XP002578694,
- R. JIN ET AL.: "Enzyme-mediated fast in situ formation of hydrogels from dextran-tyramine conjugates", BIOMATERIALS, vol. 28, 25 February 2007 (2007-02-25), pages 2791-2800, XP002578695,

## Description

### FIELD OF THE INVENTION

The invention is in the field of tissue engineering. More specifically, the invention is in the field of polymers which can be used in the preparation of injectable hydrogels. More in particular, the invention relates to dextran-hyaluronic acid conjugates, methods for producing them, and uses thereof.

### BACKGROUND OF THE INVENTION

Tissue engineering represents a promising approach in the treatment of damaged cartilage. This approach generally involves the use of three-dimensional (3-D) scaffolds, which can support the growth, proliferation and differentiation of incorporated chondrocytes and/or progenitor cells. Because hydrogels are 3-D elastic networks having high water content, they mimic hydrated native cartilage tissue and are considered suitable scaffolds for cartilage tissue engineering. Injectable hydrogels are highly desirable in clinical applications since they can be applied via a minimally invasive procedure.

After injection in the form of a solution, the precursor gels in situ and fills the irregularly shaped defect. Meanwhile, cells and/or bioactive molecules can be easily incorporated. Injectable hydrogels can be obtained via a chemical crosslinking method, for example, photopolymerization. In this approach, a solution of a vinyl-containing polymer converts into a gel by exposure to visible or ultraviolet light in the presence of photo-initiators. Photo-crosslinked hydrogels generally have a short gelation time and are chemically stable and mechanically strong. However, cytotoxic photo-initiators and UV light required for photopolymerization reaction may induce cell death. In addition, the reaction may be exothermic, which may harm the incorporated cells and induce local necrosis. Alternatively, injectable hydrogels can be generated via Michael type addition reactions of thiol groups to (meth)acrylate, (meth)acrylamide, or vinyl sulfone groups. In this approach, thiol-bearing bioactive molecules such as adhesion peptides and matrix metalloproteinase substrate peptides can be relatively easily incorporated creating biomimetic hydrogels. However, the pace of gelation induced by a Michael type addition reaction, in general, was found to be too slow (∼30 min or longer), which hampers clinical applications. Recently, an enzymatic crosslinking method using peroxidase, which induces fast gelation, has been developed. Dextran- and chitosan-based injectable hydrogels based on this approach are known in the art. Crosslinking takes place via an oxidative coupling reaction of phenol moieties in the presence of horseradish peroxidase (HRP) and H₂O₂. These hydrogels were formed rapidly within minutes. They showed good biocompatibility and support chondrocyte survival and differentiation (R Jin et al. Biomaterials 2009;30: 2544-2151 and R Jin et al. Journal of Controlled Release 2008; 132: e24-e6).

R. Jin et al. Biomaterials 2007;28: 2791-2800 discloses enzymatic cross-linking of dextran-tyramine conjugates and the rheological, swelling and degradation properties of the obtained hydrogels.

However, there is a need in the art to provide hydrogels which are crosslinked enzymatically, that have a better degradability, a higher storage modulus, a better stability, improved biocompatibility, improved performance in tissue regeneration and a better swelling behavior. It is an objective of the present invention to provide the means to prepare hydrogels which fulfill at least one of these needs.

### SUMMARY OF THE INVENTION

The invention provides a copolymer of hyaluronic acid (HA) grafted with a dextran-tyramine (Dex-TA) conjugate. In a preferred embodiment said dextran-tyramine conjugate has a degree of substitution (DS), defined as the number of conjugated tyramine moieties per 100 anhydroglucose rings between 5 and 25. Preferably, the dextran chain in said dextran-tyramine conjugate has an average molecular weight between 10 and 80 k. Preferably, said copolymer according to claim 1 is for use as a medicament, preferably for the treatment of a subject in need of an implant.

In another aspect, the invention further provides a method for preparing a copolymer comprising steps of
modifying a dextran-tyramine conjugate at the reducing terminal glucose residue with an excess of N-Boc-1,4-diaminobutane;
subjecting said modified dextran-tyramine conjugate to reductive amination using sodium cyanoborohydride;
deprotecting the Boc group using trifluoroacetic acid to obtain a conjugate with a terminal free primary amine group (denoted as Dex-TA-NH₂).

In another aspect the invention further provides a composition comprising a copolymer according to the invention with a suitable amount of hydrogen peroxide and suitable amount of a peroxidase. Preferably, the amount of said copolymer is between 5 and 30 wt %. Preferably, the amount of peroxidase is between 0.1-10 Units/mL. Preferably, the concentration of hydrogen peroxide is between 0.001 and 0.01 M. Preferably, said composition is an injectable hydrogel. Preferably, said composition further comprises cells, preferably stem cells and or chondrocytes, wherein said stem cells are more preferably mesenchymal stem cells, embryonic stem cells or pluripotent stem cells or combinations of any of these cells.

Preferably, said composition is used as a medicament, preferably for the treatment of a subject in need of an implant.

In another aspect, the invention further provides a method of treating a subject by providing an injectable hydrogel according to the invention.

### DESCRIPTION OF THE DRAWINGS

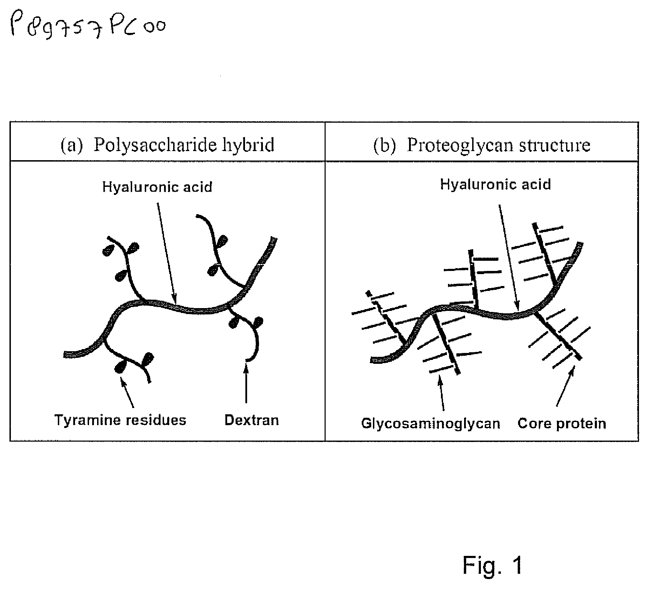

Figure 1 shows the chemical structure of (a) polysaccharide hybrids based on hyaluronic acid and dextran-tyramine conjugates and (b) structure of a proteoglycan.
Figure 2 shows the synthesis of hyaluronic acid grafted with dextran-tyramine conjugates (HA-g-Dex-TA)
Figure 3 shows the 1H-NMR spectra of dextran, Dex-TA, Dex-TA-NH-Boc and Dex-TA-NH2; (b) hyaluronic acid (HA) and HA grafted with dextran-tyramine conjugates (HA-g-Dex-TA) in D₂O.
Figure 4 shows GPC chromatograms of Dex-TA-NH2 DS 10, HA and the copolymer HA-g-Dex-TA DS 10. Eluent: NaAc buffer (300 Mm, pH 4.5, containing 30% (v/v) methanol).
Figure 5 shows gelation times of hydrogels based on HA-g-Dex-TA as a function of DS and concentration. (** p<0.01)

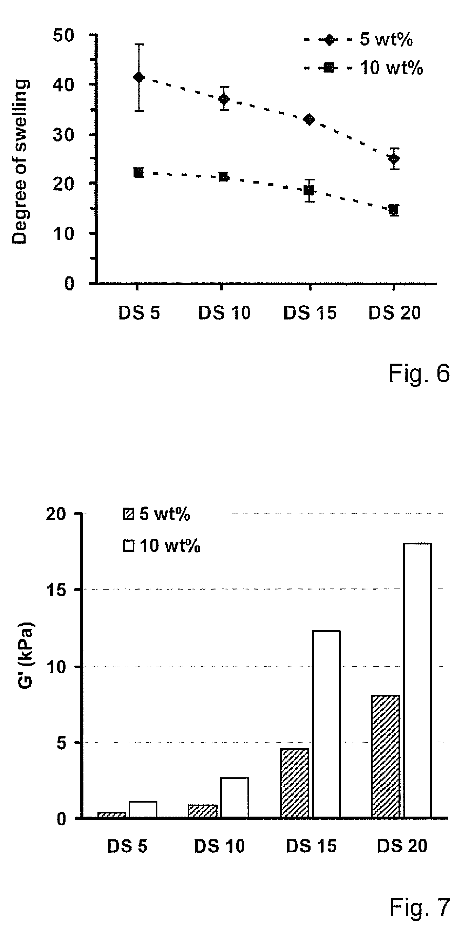

Figure 6 shows the degree of swelling of HA-g-Dex-TA hydrogels as a function of DS.
Figure 7 shows the storage modulus of HA-g-Dex-TA hydrogels as a function of DS.
Figure 8 shows the enzymatic degradation of HA-g-Dex-TA hydrogels at DS 5, 10 (a) and DS 15, 20 (b) exposed to PBS containing 100 U/ml HAse at 37 °C.
Figure 9 presents a Live-dead assay showing chondrocytes incorporated in HA-g-Dex-TA DS 15 (A and C) and DS 20 (B and D) hydrogels after 7 (A and B) and 14 (C and D) days in culture. Scale bar: 500 µm.
Figure 10 shows SEM images of chondrocytes incorporated in the (a) HA-g-Dex-TA DS 15 and (b) HA-g-Dex-TA DS 20 hydrogels at day 21. High magnification SEM images of the boxed regions of Figure 9a and 9b are shown in Figure 9c and 9d, respectively.
Figure 11 shows (a) swelling and (b) degradation of Dex-TA and HA-g-Dex-TA hydrogels in the presence of chondrocytes as a function of culturing time. (* p<0.05, ** p<0.01, *** p<0.001, vs. Dex-TA DS 15; $ p<0.05, vs. Day 1; † p<0.01, vs. Day 7)

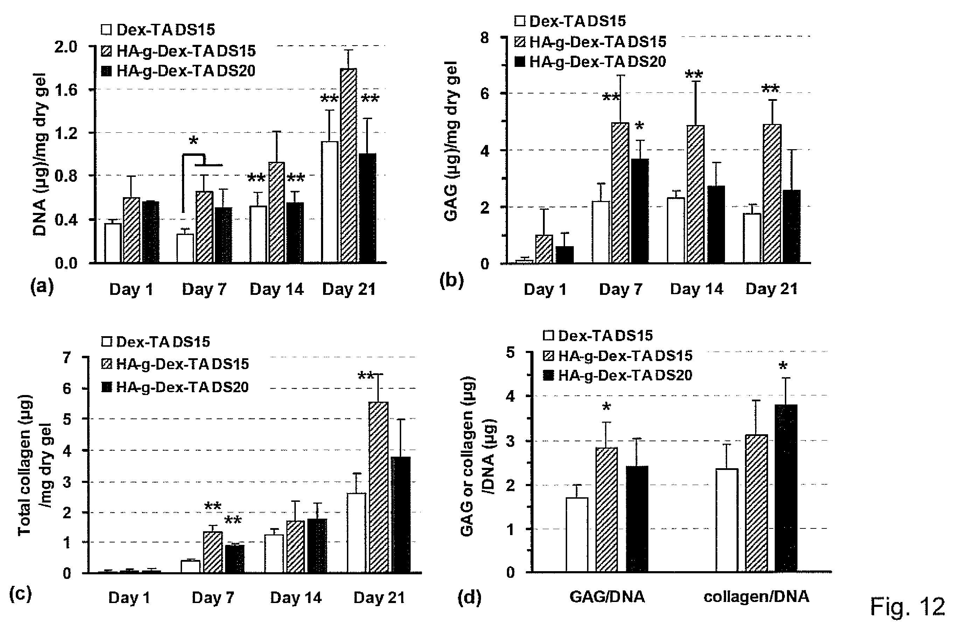

Figure 12 shows (a) the DNA content normalized to dry gel weight of Dex-TA DS 15, HA-g-Dex-TA DS 15 and 20 hydrogels containing chondrocytes after in vitro culturing for 1, 7, 14 and 21 days. (* p<0.05 at day 7; ** p<0.01 vs. HA-g-Dex-TA DS 15 at day 14 and 21) (b) GAG and (c) total collagen accumulation (values were normalized to the dry gel weight per sample) in Dex-TA and HA-g-Dex-TA hydrogels containing chondrocytes after in vitro culturing for 1, 7, 14 and 21 days. (** p<0.01 vs. Dex-TA DS 15 at each time point) (d) GAG and total collagen content normalized to DNA content in Dex-TA and HA-g-Dex-TA hydrogels containing chondrocytes after in vitro culturing for 21 days. (* p<0.05 vs. Dex-TA DS 15)
Figure 13 shows the subcutaneous implantation of an *in situ* gelating Dex-HA hydrogel in immuno-competent mice (A) or injected with the polymer solution without crosslinking as a control (B). Samples were explanted after 3, 7 and 28 days. Representative sections of 3 µm were stained with H&E.

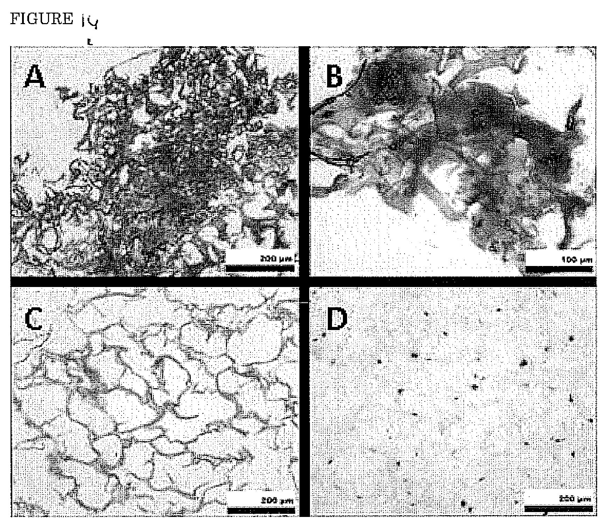

Figure 14 shows the histological analysis of cartilage formation in subcutaneous implantated Dex-HA hydrogels in comparisson with Dex-TA hydrogels in nude mice. Samples were explanted after 28 days and subjected to histochemistry. Representative sections of 10 µm were stained with Safranin O, which stains glycosaminoglycans red. A) Efficient cartilage formation in implanted Dex-HA hydrogels with incorporated chondrocytes (10 million cells/ml). B) Higher magnification of A showing the presence of cells. The porous hydrogel network is almost completely filled with cartilaginous matrix. C) Dex-HA hydrogels implanted without cells. Hardly any cartilage formation is visible and the gel retains its porous structure. D) Cartilage formation in Dex-TA hydrogels with incorporated chondrocytes (10 million cells /ml). Some cartilage formation is noted particularly surrounding the incorporated chondrocytes. Cartilage formation is strongly reduced in comparison to the Dex-HA hydrogels (compare with figure A and B). In addition, the hydrogel network still contains an open network structure.

### DETAILED DESCRIPTION OF THE INVENTION

### Definitions

The term 'treatment of a patient in need of an implant' as used herein refers to a treatment aiming to restore or to replace the function of a missing tissue and wherein the provision of the hydrogel of the invention is aimed at improving regeneration of a damaged tissue wherein said implant is implanted. In other embodiments, the treatment is aimed at the sustained or extended release of a medicament or drug incorporated in said hydrogel. Preferably, said missing tissue is cartilaginous tissue. Preferably said sustained or extended release refers to slow release which is at least 3, 4, 5, 6, 8, 10, 12 15, 20, 24 hours. In some embodiments said sustained or extended release is at least 1, 2, 3, 4, 5, 6, 7, 10, 14, 21 days. Preferably, said slow or sustained release is at least prolonged when compared to the same medicament not incorporated in a hydrogel of the invention, preferably by at least 3, 4, 5, 6, 8, 10, 12 15, 20, 24 hours. Preferably, said subject is suffering from a tissue defect, preferably a cartilage defect. Said subject is preferably a mammalian animal, preferably a human.

The term "hydrogel" as used herein refers to three-dimensional hydrophilic polymeric networks. Hydrogels have high water content, providing an environment similar to native cartilage. Besides, hydrogels allow for sufficient transportation of nutrients and waste products, which is essential for cell growth. Hydrogels are preferably designed such that they will form in-situ and these systems are termed injectable hydrogels. They offer the advantages of good alignment with irregularly shaped defects and allow easy incorporation of cells or biologically active factors such as growth factors or drugs. Moreover, from the clinical point of view, implantation surgery can be avoided and replaced by a simple injection procedure.

The term "injectable hydrogel"refers to a solution which is capable of forming a hydrogel once it has been injected. Therefore, said solution is fluid enough to enable injection of said fluid.

The term "Dextran-tyramine" as used herein refers to a dextran molecule grafted with tyramine molecules linked by a urethane bond or by an ester-containing diglycolic group. It is contemplated that also other molecules containing phenol groups may be used for the coupling to dextran.

The term "growth factor" as used herein refers to a molecule that elicits a biological response to improve tissue regeneration, tissue growth and organ function. Preferred growth factors are morphogens. The term 'morphogen' as used herein refers to a substance governing the pattern of tissue development and, preferably, the positions of the various specialized cell types within a tissue. Preferably, it spreads from a localized source and forms a concentration gradient across a developing tissue.

In preferred embodiments, a morphogen is a signaling molecule that acts directly on cells (preferably not through serial induction) to produce specific cellular responses dependent on morphogen concentration. Preferred morphogens include: a Decapentaplegic / Transforming growth factor beta (TGFbeta), Hedgehog / Sonic Hedgehog, Wingless / Wnt, an Epidermal growth factor (EGF), a Bone Morphogenic Protein (BMPs), and a Fibroblast growth factor (FGF). Preferably, said FGF comprises FGF2, KFG and FGF18. Preferably, said BMP comprises BMP2, BMP4, BMP6 and BMP7. Preferred TGFbeta's include TGFbeta1 and TGFbeta3. In some preferred embodiments, said growth factor comprises a protein of the extracellular matrix.

The term "growth factor antagonist" as used herein refers to secreted growth factor antagonists (BMP antagonists (noggin, gremlin), Wnt-antagonists (Dkk1, FrzB, sclerostin) and dual antagonists of both BMP and Wnt (Cerberus).

The term "tyramine conjugate" in aspects of the invention includes reference to conjugates comprising additional moieties conjugated to the primary conjugate. Examples thereof include heparin conjugated to Dex-TA.

The term "suitable amount" with respect to peroxidase or peroxide as referred to herein, means a curing amount said substance, which refers to the ability to amount to gel formation in a composition of the invention. The gel formation may be established by the methods as referred to in the examples using the tilting method.

When reference is made herein to peroxide and peroxidase, these terms should be understood as referring to a curing system for crosslinking the tyramine conjugates in compositions of the invention. The skilled artisan will understand that alternative curing systems based on curing radicals, including oxygen radicals, may be used in aspects of the inventions and are envisioned as embodiments in aspects of the invention.

### Description of the embodiments

The present invention is based on the finding that hydrogels based on polysaccharide hybrids (HA-g-Dex-TA) have highly advantageous characteristics which make them especially suitable for application as injectable hydrogels. Hydrogels based on polysaccharide hybrids (HA-g-Dex-TA) have a short gelation time (less than 2 minutes) which can be adapted by varying the degree of substitution and/or the copolymer concentration. Furthermore, these hydrogels are readily degraded in the presence of hyaluronidase and have a high storage modulus, an advantageous swelling behaviour and an adequate stability.

The behaviour of chondrocytes incorporated inside HA-g-Dex-TA hydrogels demonstrated that the gel systems had a good biocompatibility. Compared to Dex-TA hydrogels, these biomimetic HA-g-Dex-TA hydrogels induced an enhanced cell proliferation and matrix deposition (increased glycosaminoglycan and collagen production). As a result, hydrogels based on polysaccharide hybrids (HA-g-Dex-TA) enable more cartilage formation when compared to hydrogels based on Dextran Tyramine conjugates, as is demonstrated herein. In conclusion, the present invention shows that these novel injectable biomimetic hydrogels based on polysaccharide hybrids are very advantageous for the development of scaffolds for cartilage tissue engineering.

Therefore, the invention provides a copolymer of hyaluronic acid (HA) grafted with a dextran-tyramine (Dex-TA) conjugate.

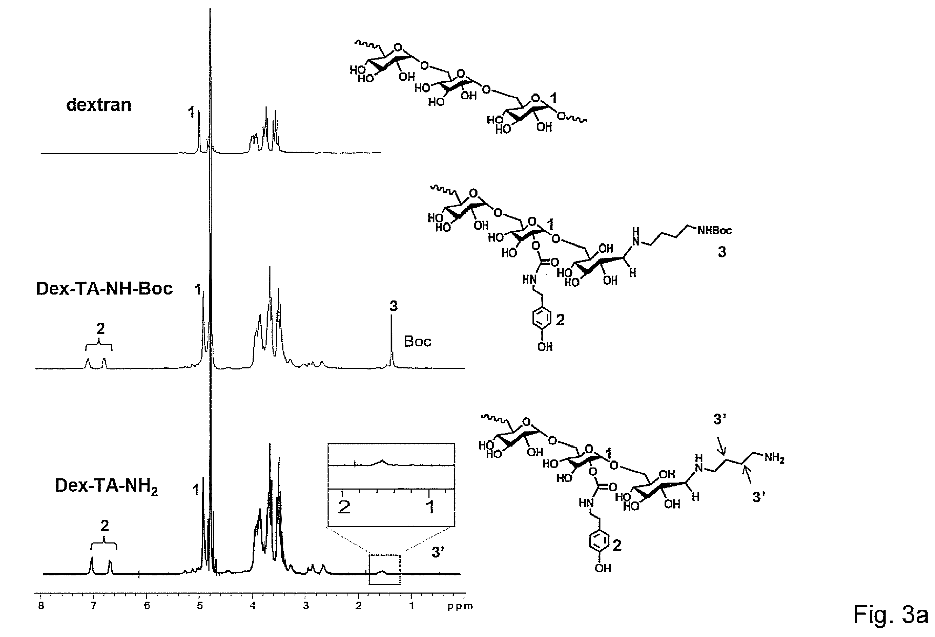

Dextran-tyramine conjugates are known in the art (R Jin et al. Journal of Controlled Release 2008;132: e24-e6). Preferably, said dextran-tyramine conjugate has a degree of substitution (DS), defined as the number of conjugated tyramine moieties per 100 anhydroglucose rings between 5 and 25. The degree of substitution can be determined using 1H NMR by comparing the integrals of signals at δ 5.0 (see anomeric protons, Figure 3a, peak 1) and δ 6.9-7.2 (aromatic protons, Figure 3a, peak 2). Different preferred Dex-TA conjugates with DS values of between 5 and 20 can be prepared by changing the feed molar ratio of p-nitrophenyl chloroformate to hydroxyl groups in dextran from 0.05 to 0.25. In some preferred embodiments, said copolymer of the invention is a copolymer wherein the dextran chain in said dextran-tyramine conjugate has an average molecular weight between 5 and 80 kD, more preferably between 10 and 80 kD. A skilled person can easily alter the molecular weight of said copolymer by preparing Dextran-TA having longer or shorter dextran chains.

Medical application of the copolymers is therefore within the scope of the invention. More specifically, the medical use of said copolymer in the field of tissue engineering belongs to the scope of the invention.

### Preparation of Dex-TA conjugates

Dex-TA conjugates can suitably be prepared by functionalizing dextran with tyramine moieties to give the Dex-TA conjugate. Said dextran preferably has a molecular weight between 5 and 80 kDa, more preferably between 10 and 80 kDa. Preferably said dextran has a molecular weight higher than 5, 10, 14, 15, 20, 25, 30, 35, 40, 45, 50, 55, 56, 57, 58, 59 kDa. Preferably, said dextran has a molecular weight lower than 75, 70, 65, 64, 63, 62, 61 kDa.

Preferably, the molecular weight (MW) of dextran in the copolymer according to the invention is between 5 and 80kD, preferably between 10 and 80 kD..

In some embodiments, the molecular weight (MW) of dextran is between 10-20 kD, preferably around 14kD, to prepare hydrogels. Even more preferred is a copolymer according to the invention for the preparation of a said hydrogel, wherein the degree of substitution of tyramine units is between 5 and 20, and more preferably in between 10-15. An advantage of said hydrogels is that they have excellent characteristics for supporting cells.

In another preferred embodiment of said copolymer, said dextran has a molecular weight between 20-40 kD, preferably around 31kD. It is even more preferred if the degree of substitution is in between 10-25. An advantage is that said hydrogels are stronger and have a molecular structure which is very suitable for storing molecules, such as growth factors, that are slowly released by the hydrogel.

The conjugates are subsequently modified at their reducing terminal glucose residue with an excess of N-Boc-1,4- diaminobutane, followed by reductive amination using sodium cyanoborohydride for several days. After the deprotection of the Boc group using trifluoroacetic acid, conjugates with a terminal free primary amine group (denoted as Dex-TA-NH₂) are obtained. Complete deprotection of the Boc group can be confirmed by 1H NMR. The obtained amine-terminated Dex-TA (denoted as Dex-TA-NH₂) can be purified by ultrafiltration and subsequently freeze-dried.

### Synthesis and characterization of copolymers HA-g-Dex-TA

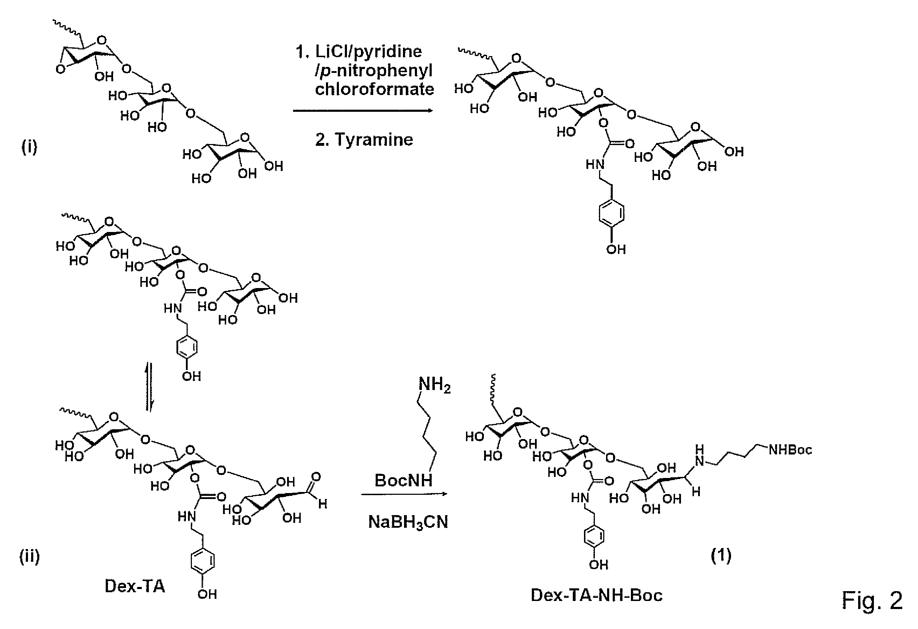

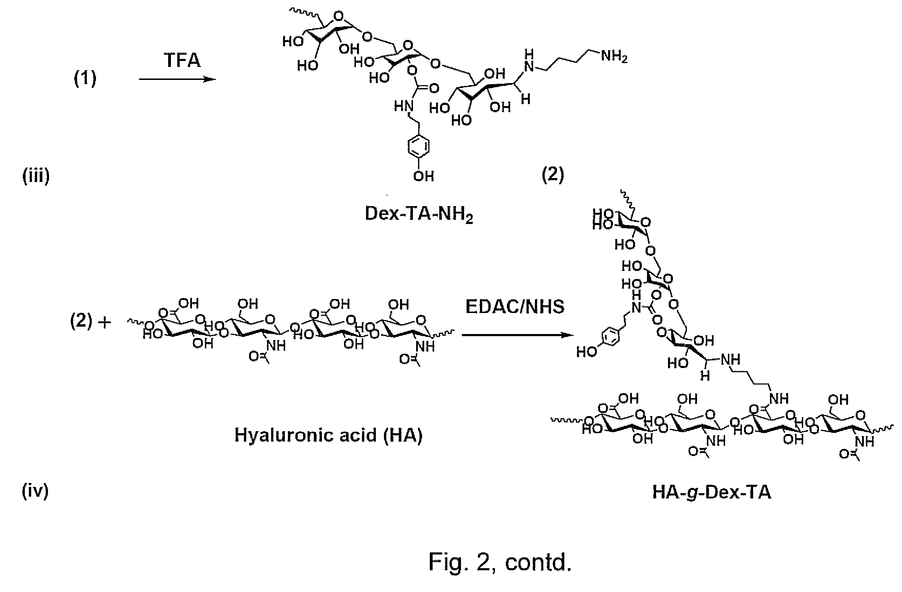

Hyaluronic acid (HA) can be grafted with said dextran-tyramine (Dex-TA) conjugate, preferably via a four step reaction, as shown in Figure 2. Finally, a coupling reaction between the primary amine groups of these Dex-TA-NH₂ conjugates and the carboxylic acid groups of HA using an EDAC/NHS activation reaction, preferably at a feed molar ratio of HA to NH₂ between 1:4 to 1:8, more preferably between 1:5 and 1:7 and most preferably at a feed molar ratio of 1:6, result in the HA-g-Dex-TA graft copolymers according to the invention. The molecular weights of these polymers can be determined by gel-permeation chromatography (GPC).

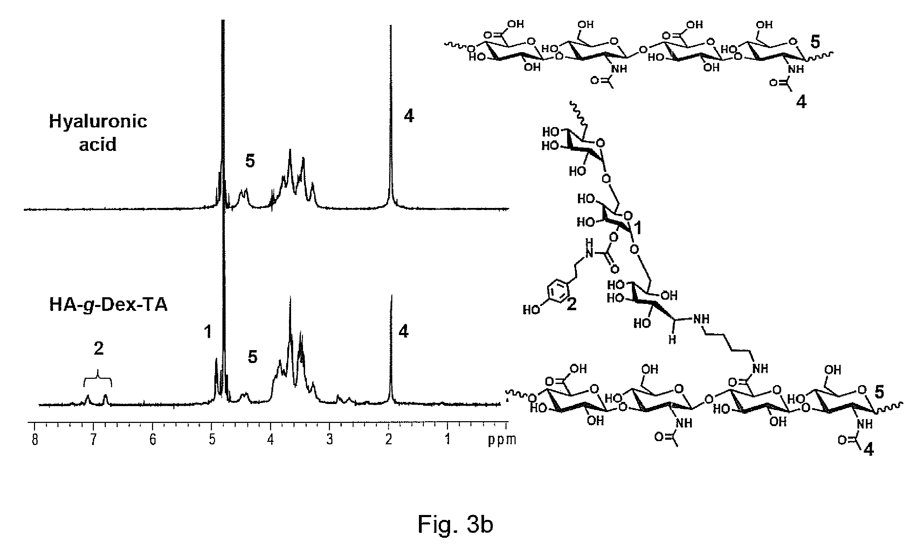

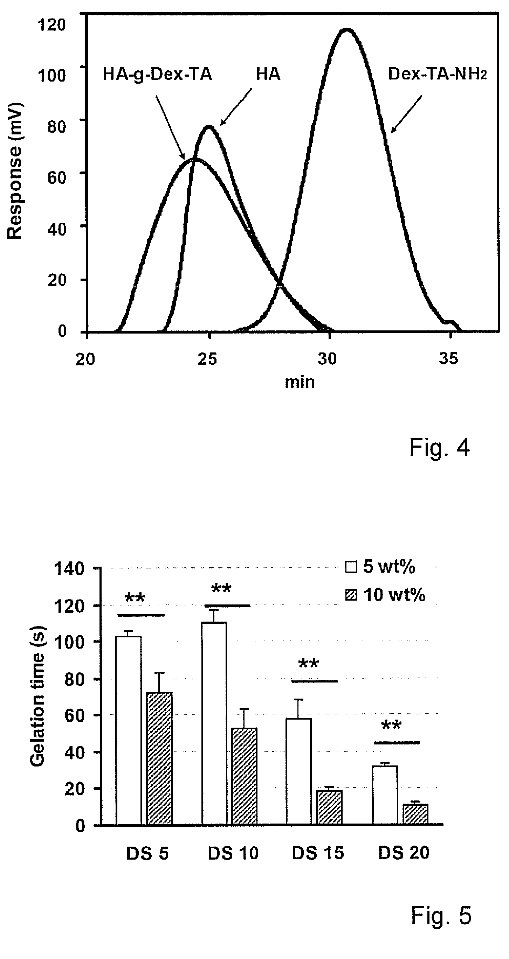

Typical elution profiles of Dex-TA-NH₂ DS 10, HA and HA-g-Dex-TA DS 10 are presented in Figure 4. The HA-g-Dex-TA DS 10 polymer was eluted earlier than HA and Dex-TA-NH₂ DS 10 in a unimodal GPC-trace. This indicates that the HA-g-Dex-TA polymer is successfully synthesized. The average number molecular weights of these polymers thus formed ranges from 38.0 to 40.0 kg/mol with a relatively low polydispersity index (PDI 1.3-1.7) (Table 1). The chemical structure of the HA-g-Dex-TA polymers can be confirmed by 1H NMR. In Figure 3b, it is shown that besides signals attributable to the anomeric and methyl protons of HA (peaks 4 and 5), new peaks at 5.0 (peak 1) and 6.9-7.2 (peaks 2) are present in the spectra. These peaks are attributable to the anomeric and aromatic protons from coupled Dex-TA. The number of grafted Dex-TA conjugates per HA molecule is approximately 4, as determined with 1H NMR by comparing the integrals of signals at δ 2.0 (methyl protons of acetamide groups in HA) and δ 5.0 (dextran anomeric protons) (Table 1). It was found that the average number molecular weights of the HA-g-Dex-TA polymers calculated using 1H NMR are in agreement with those determined by GPC measurements.

### Hydrogel formation and gelation time

The invention further provides a composition comprising a copolymer according to the invention with a suitable amount of hydrogen peroxide and suitable amount of a peroxidase. Hydrogels of HA-g-Dex-TA are conveniently prepared in PBS or equivalent buffers by peroxidase (preferably HRP) mediated coupling reaction of phenol moieties. Preferably, said peroxidase is present in an amount between 0.1 and 10 Units/ml. More preferably, said concentration is between 0.5 and 5, more preferably between 0.36 and 2.87 Units/ml. Gels having good cytocompatibility are obtained with 0.25 mg HRP per mmol phenol moieties and a molar ratio of H₂O₂/TA between 0.1 and 0.3, more preferably 0.2.

Preferably, the concentration of hydrogen peroxide in said hydrogel is between 0.001 and 0.01 M.

The gelation time can be determined by the vial tilting method. The enzymatic crosslinking of HA-g-Dex-TA according to the invention leads to fast gelation. The longest gelation times were found for the HA-g-Dex-TA copolymers with a low DS, due to the decreased number of tyramine units per chain.

Effects on gelation time are even more pronounced with decreasing polymer concentration. The fastest gelation, within 10 s, occurred using HA-g-Dex-TA having DS between 18 and 22, more preferably a DS of 20 hydrogels at a polymer concentration between 8 and 12 wt%, more preferably of 10 wt%. Thus, an attractive feature of these HA-g-Dex-TA hydrogel systems is that the gelation occurred in a reasonably short time (10 s to 2 min) under mild conditions. Preferred embodiments of composition according to the invention therefore have an amount of said copolymer between 5 and 30 wt % and more preferably between 10 and 20 wt %. Furthermore, gelation times of the HA-g-Dex-TA hydrogels can be easily tuned by adjusting the DS of tyramine units and polymer concentration, which makes the systems highly suitable as injectable scaffolds for various applications.

Preferably, said composition further comprises cells, preferably stem cells and or chondrocytes, wherein said stem cells are more preferably mesenchymal stem cells, embryonic stem cells or pluripotent stem cells or combinations of any of these cells.

Preferably, said composition further comprises a growth factor, preferably a Bone Morphogenetic Protein or a TGFbeta. In another preferred embodiment, said composition further comprises a growth factor, preferably a Bone Morphogenetic Protein or a TGFbeta. An advantage thereof is that growth factors accelerate differentiation and migration of relevant cells in the implant or in the surrounding tissue and said use increases the successful regeneration, reconstruction and replacement of lost and worn out tissues. Without wishing to be bound by theory, it is believed that as a result of the presence of said growth factor, the balance between factors that stimulate and factors that inhibit cell proliferation, cell differentiation, cell maturation, cell death and the formation of a functional organ cell growth is altered such that a better regeneration is achieved.

In another embodiment, said composition comprises a therapeutically effective medicament. Said medicament may be any pharmaceutically effective compound or biological molecule, including but not limited to a small molecule, a hormone, a growth factor, a growth factor antagonist, a peptide, a protein and an anti-cancer drug.

### Hydrogel characterization

The values of swelling are lower at higher DS values of tyramine units and higher polymer concentration. This can be explained by the increased crosslinking density of the hydrogels. Compared to Dex-TA hydrogels, HA-g-Dex-TA hydrogels display an improved swelling behaviour. Without being bound by theory, it is believed that this can be explained by an increased water uptake resulting from the electrostatic repulsion of negatively-charged HA chains at pH 7.4.

Hydrogels prepared at a concentration of 10 wt% showed a 2 to 3 fold higher storage modulus compared to 5 wt% hydrogels. Furthermore, by increasing the DS from 5 to 20, the corresponding G' values significantly increased. This is most likely due to the increased crosslinking density in DS 10 gels versus DS 5 gels. In general, the moduli of HA-g-Dex-TA hydrogels range from 370 to 18000 Pa. This is comparable to values previously reported for dextran-tyramine hydrogels. They are, however, much higher than values reported for other enzymatically-crosslinked hydrogels such as hyaluronic acid-tyramine DS 6 hydrogels (10-4000 Pa) (F Lee et al. Soft Matter 2008;4: 880-7).

### Enzymatic degradation

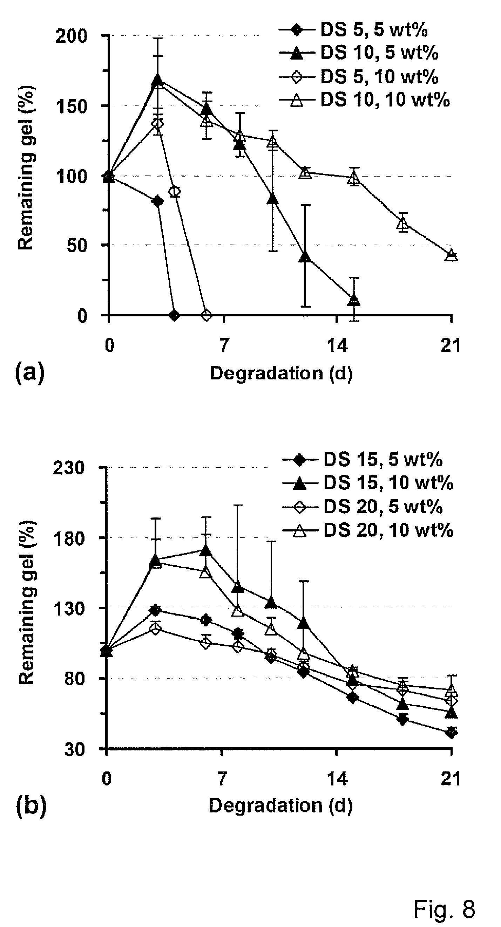

The inventors have determined that the gels made using the compositions according to the invention are biodegradable via enzymatic hydrolysis using hyaluronidase (HAse). In the presence of hyaluronidase, the gel weight first increased because of swelling and degradation, and then decreased due to degradation and dissolution of small fragments. The degradation time as used herein is defined as the time required to completely dissolve at least one of 3 samples tested. It was found that the HA-g-Dex-TA hydrogels swelled initially and then degraded at rates that depend on DS and polymer concentration (Figure 8). The HA-g-Dex- TA DS 5 hydrogels prepared at polymer concentrations of 5 and 10 wt% were completely degraded after 4 and 6 days, respectively, while the 5 wt% HA-g-Dex-TA DS 10 hydrogels showed a longer degradation time of 15 days. The hydrogels of HA-g-Dex-TA at a high DS of 15 and 20 were more stable with more than 30 wt% of gel remaining after 21 days of degradation. Even after 2 months, these gels were not completely degraded. Compared to previously reported hyaluronic acid-tyramine (HA-TA) hydrogels which were completely degraded within 1 day in the presence of 25 U/mL of HAse in PBS, hydrogels according to the invention are much more stable even in the presence of 4-fold higher 15 concentrations of HAse (100U/mL) with prolonged degradation times. Therefore, in some preferred embodiments, the amount of HA-g-Dex-TA in said hydrogel is preferably between 20 and 50 wt%, more preferably between 25 and 30 wt%. The increased stability is attributed to the presence of dextran, which probably stabilized the hydrogels. The improved degradation characteristics compared to HA-TA gels makes HA-g-Dex-TA hydrogels more suitable for cartilage tissue engineering.

### Cytotoxicity

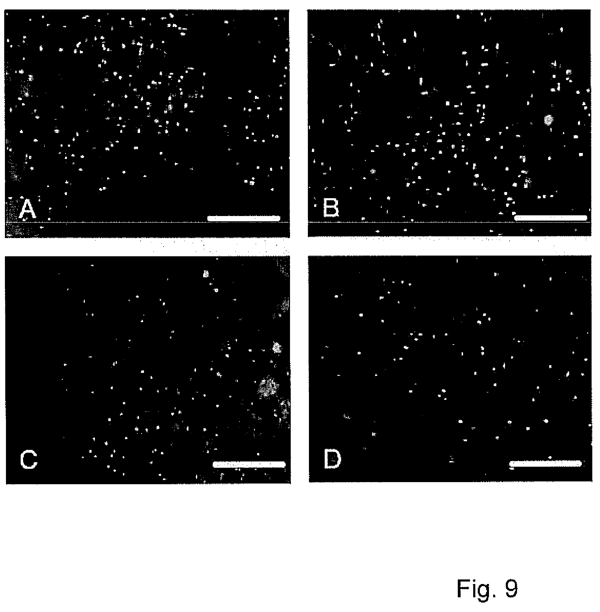

In cell experiments, hydrogels based on compositions according to the invention comprising between 8 and 12 wt% HA-g-Dex-TA, more preferably 10 wt% HA-g-Dex-TA with a degree of substitution (DS) between 15 and 20 showed best stability. These hydrogels were selected for the preparation of gel/cell constructs for in vitro studies. The cytocompatibility of HAg- Dex-TA DS 15 and 20 hydrogels was investigated by the incorporation of bovine chondrocytes in HAg- Dex-TA hydrogels at a polymer concentration of 10 wt%. Cell survival of the chondrocytes was analyzed by a Live-dead assay (Figure 9), in which living cells fluoresce green and dead cells fluoresce red. In both HA-g-Dex-TA DS 15 and DS 20 hydrogels, over 95% of chondrocytes stained green, indicating cytocompatible enzymatic crosslinking conditions.

### Chondrocyte morphology

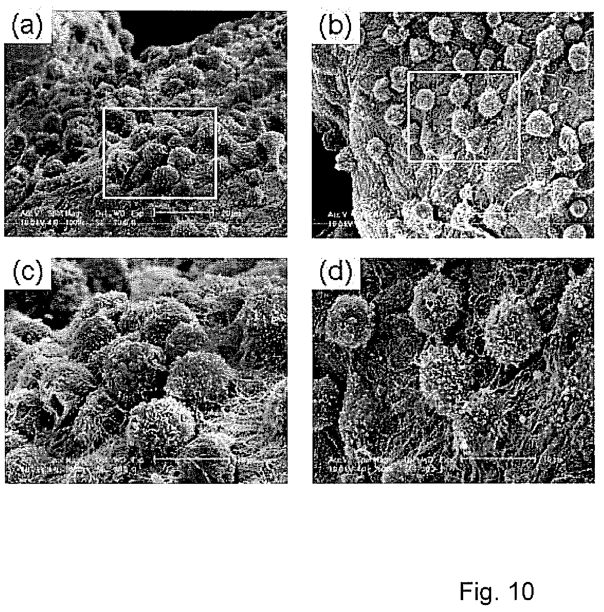

The cell/scaffold constructs were investigated by SEM (Figure 10). The chondrocytes encapsulated inside HA-g-Dex-TA DS 15 and 20 hydrogels retained a round shape at 21 days in culture, which was also observed in Dex-TA DS 15 hydrogels. High magnification of SEM images showed that the chondrocytes deposited an extracellular matrix.

### Swelling and degradation of hydrogels in the presence of chondrocytes

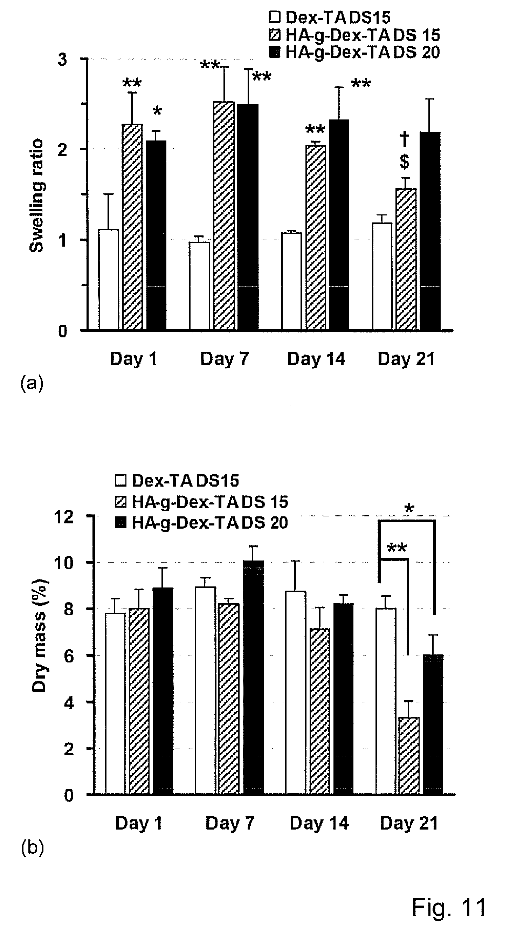

To study the swelling and degradation behaviour of the HA-g-Dex-TA hydrogels in the presence of chondrocytes, the constructs were incubated in a chondrocyte expansion medium and weighed at regular intervals. The swelling ratio of a Dex-TA DS 15 hydrogel remained almost constant during the total 16 culturing time up to 21 days. In contrast, the swelling ratios of the HA-g-Dex-TA hydrogels increased from day 1 to day 7 and decreased slightly after day 14 (Figure 11a). The swelling behaviour suggested a loss in crosslinking density with time as a result of degradation. This is supported by the pronounced decrease in the swelling ratio for HA-g-Dex-TA DS 15 hydrogels at day 14 and day 21 compared to day 1. The degradation of HA-g-Dex-TA hydrogels was further studied by the determining the percentage of dry gel mass of the hydrogels (Figure 11b). Dex-TA DS 15 hydrogels had a dry gel mass of 8% at day 1, which remained stable up to 21 days. In contrast, the values for HA-g-Dex-TA DS 15 and 20 hydrogels decreased from 8% at day 1 to 3% and 6% at day 21, respectively. The significant differences between the Dex-TA and the HA-g-Dex-TA hydrogels are most likely explained by the expression of hyaluronidase by incorporated chondrocytes.

### Cell proliferation and matrix production

Chondrocyte proliferation in HA-g-Dex-TA DS 15 and DS 20 hydrogels was assessed by a CyQuant DNA assay by measuring DNA content of the hydrogels during the culturing period up to 21 days. The phenotype of chondrocytes incorporated was characterized in terms of their matrix production. The ECM matrix produced was analyzed by a dimethylmethylene blue assay and a hydroxyproline assay for glycosaminoglycans (GAGs) and collagen, respectively, and the values were normalized to the dry gel weight of each sample. In the CyQuant DNA assay, the DNA content was expressed as the DNA amount normalized to the dry gel weight. Results were compared to hydrogels prepared from a dextran-tyramine conjugate Dex- TA DS 15 (Mn, Dex=14.5 kg/mol) which served as a reference. In general, in all these hydrogels the DNA content increased with increasing culturing time. Interestingly, at day 21, the DNA content in the HA-g-Dex-TA DS 15 hydrogel was higher than that in the Dex-TA DS 15 hydrogel (Figure 12a). These results demonstrated the superiority of HA-g-Dex-TA hydrogels over Dex-TA hydrogels. In the control group of GAG assay, hydrogels prepared from Dex-TA DS15, the GAG content increased from day 1 to day 7, and then remained unchanged throughout the experimental period (Figure 12b). Similar trends were also observed for the HA-g-Dex-TA hydrogels. The HA-g-Dex-TA DS 15 hydrogels showed a statistically higher average GAG content per mg of dry gel weight than the Dex-TA DS 15 hydrogels at day 14 and 21 (4.8 vs 2.3 and 4.9 vs 1.7, respectively). The average GAG content in HA-g-Dex-TA DS 20 hydrogels was found to be 2.7 and 2.6 µg per mg of dry gel weight at day 14 and 21, respectively, which was close to that of Dex-TA DS 15 hydrogels. These results suggest that the degree of substitution of the Dex-TA conjugate in the HA-g-Dex-TA had a direct effect on the GAG production, indicating that appropriate design of the gel chemistry might lead to a better performance in ECM production. The total collagen content, determined using a hydroxyproline assay, was normalized to the dry gel weight (Figure 12c). The results showed that the total collagen accumulation increased in time and reached the highest value at day 21 for all groups. Interestingly, the average value of total collagen content was higher in HA-g-Dex-TA hydrogels having DS between 15 and DS 20 than in Dex-TA DS 15 hydrogels, irrespective of the culturing time. After 7 and 21 days in culture, significant higher collagen deposition was found in HA-g-Dex-TA DS 15 than in Dex-TA DS 15 hydrogels. For comparative studies, the GAG and total collagen content were normalized to the DNA content, as shown in Figure 12d. In general, enhanced matrix deposition was observed for HA-g-Dex-TA compared to Dex-TA hydrogels. At day 21, the GAG/DNA ratio was 1.6 fold higher in the HA-g-Dex-TA DS 15 hydrogels than Dex-TA hydrogels. Further, significantly higher collagen/DNA ratios were observed for HA-g-Dex-TA DS 20 hydrogels than for Dex-TA DS 15 hydrogels. Taken together, hydrogels based on HA-g-Dex-TA hybrids showed a better stability than HA-TA hydrogels and an improved chondrocyte performance than Dex-TA hydrogels. Thus they are superior as injectable scaffolds for cartilage tissue engineering.

In a preferred embodiment, said composition according to the invention is an injectable hydrogel. Said hydrogel can also be marketed as pre-filled syringes. The invention further provides a method of treating a subject by providing a composition or an injectable hydrogel according to the invention.

In another aspect, the invention further provides a method of treating a subject in need of tissue repair by providing a composition or hydrogel according to the invention. Said composition or hydrogel may be used in a monotherapy (i.e. use of the hydrogel, composition alone). Alternatively, the composition or hydrogel according to the invention may be used as an adjunct, or in combination with other known therapies.

Preferably, the composition or hydrogel according to the invention may be administered by injection into an area where an implant is required for replacing a tissue. In another embodiment, said composition or hydrogel is administered to a tissue where sustained release of a medicament is required. Preferably said hydrogel is formed in situ in said area. Preferably, said hydrogel is formed within 2 hours, more preferably within 90, 80, 70, 60, 50, 40, 30, 20, 10, 9, 8, 7, 6, 5, 4, 3, 2, 1 minute(s). In some embodiments, said injection may be intravenous (bolus or infusion) or subcutaneous (bolus or infusion).), for example for closing a vessel.

In some embodiments, cells (such as chondrocytes, fibroblasts, osteoblasts, osteoclasts, mesenchymal stem cells, stem cells (not from human embryos) or a biopt and other cells as described herein etc.), medicaments, growth factors, scaffolds or other factors as described herein are provided separately from said composition or hydrogel according to the invention or mixed therewith in situ. In a preferred embodiment, said composition or hydrogel is administered intratumorally. More preferably, said hydrogel formed in the tumor contains an antitumor medicament, including but not limited to IL-2.

It will be appreciated that the amount of composition or hydrogel according to the invention required will be determined by for example the volume of said area where a tissue is to be replaced, but in addition to the swelling behaviour and degradation characteristics of said hydrogel and whether the composition or hydrogel is being used as a monotherapy or in a combined therapy. If said treatment is also used to provide slow release of a medicament, then the amount is also dependent on de dose of a medicament incorporated in said hydrogel and the pharmacokinetics of said medicament.

Optimal dosages to be administered may be determined by those skilled in the art, and will vary with the particular medicament in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. in vivo experimentation, clinical trials, etc.), may be used to establish specific formulations of the medicament according to the invention, and precise therapeutic regimes (such as daily doses and the frequency of administration).

For slow release applications, generally a daily dose of between 0.01 µg/kg of body weight and 1.0 g/kg of body weight of the hydrogel according to the invention may be used for the prevention and/or treatment of the specific medical condition. More preferably, the daily dose is between 0.01 mg/kg of body weight and 100 mg/kg of body weight. Daily doses may be given as a single administration. Alternatively, the medicament may require administration twice or more times during a day.

As an example, the medicament according to the invention may be administered as two (or more depending upon the severity of the condition) daily doses of between 25 mg and 5000 mg. A patient receiving treatment may take a first dose upon waking and then a second dose in the evening (if on a two dose regime) or at 3 or 4 hourly intervals thereafter. Alternatively, a slow release device may be used to provide optimal doses to a patient without the need to administer repeated doses.

In some embodiments, especially wherein said medicament is a peptide or a protein, administration is preferably less frequent, ranging from twice a week up to once per three months or even more preferably as single doses. Some embodiments, said administration is once a week, once every two weeks, once every three weeks, once per month, once per two months.

The invention is now illustrated in a non-limiting manner by the following examples.

**Table 1. Composition, molecular weight and distribution of HA-g-Dex-TA copolymers^{a}**

| **Polymer (code)** | **M_{n,GPC} (kg/mol)** | **PDI** | **Mₙ,NMR (kg/mol)** | **Number of grafted Dex-TA per HA chain (n)** | **Number of TA per HA chain** |
|---|---|---|---|---|---|
| Dextran | 3.3 | 1.7 | -- | - | - |
| Hyaluronic acid (HA) | 25.4 | 1.8 | -- | -- | -- |
| HA-g-Dex-TA DS 5 | 39.2 | 1.6 | 40.4 | 4.3 | 4.3 |
| HA-g-Dex-TA DS 10 | 38.4 | 1.3 | 38.2 | 4.0 | 8.0 |
| HA-g-Dex-TA DS 15 | 39.2 | 1.6 | 38.3 | 4.2 | 12.8 |
| HA-g-Dex-TA DS 20 | 40.0 | 1.7 | 40.9 | 4.5 | 18.8 |

| | | | | | |
|---|---|---|---|---|---|
| a. Coupling reactions between HA and Dex-TA-NH₂ were performed in MES with a feed molar ratio of COOH to NH₂ of 1:6. | | | | | |

### EXAMPLES

### Materials

Dextran (Mr=6000, Fluka) was dried by azeotropic distillation from dry toluene. N-Boc-1,4-diaminobutane, p-nitrophenyl chloroformate (PNC), N-ethyl-N'-(3-dimethylaminopropyl) carbodiimide 5 hydrochloride (EDAC) and sodium cyanoborohydride (NaBH3CN) were purchased from Fluka. Tyramine (TA), 4-morpholino ethanesulfonic acid (MES), trifluoroacetic acid (TFA), hydrogen peroxide (H₂O₂), pyridine (anhydrous), deuterium oxide (D₂O), phosphorus pentoxide, hyaluronidase (HAse, ∼300 U/mg), lithium chloride (LiCl) and N-hydroxysuccinimide (NHS) were obtained from Aldrich- Sigma. Horseradish peroxidase (HRP, type VI, 300 purpurogallin unit/mg solid) was purchased from Aldrich and used without further purification. Sodium hyaluronate (15-30 kg/mol, laboratory grade) was purchased from CPN Shop. N,N-Dimethylformamide (DMF) was dried over CaH₂, distilled under vacuum and stored over molecular sieves (4Å). LiCl was dried at 80 °C under vacuum over phosphorus pentoxide. All other solvents were used as received. Dextran-tyramine (denoted as Dex-TA) conjugates were prepared as reported previously [10].

### Synthesis of amine-terminated dextran-tyramine conjugates

Amine-terminated dextran-tyramine conjugates (denoted as Dex-TA-NH₂) were synthesized by a two step procedure. Dex-TA conjugates were first reacted with N-Boc-1,4-diaminobutane and sodium cyanoborohydride to end functionalize the dextran. The protecting t-butyloxycarbonyl group was removed by reaction with TFA. Typically, Dex-TA (5 g), dissolved in 25 mL of deionized water, was treated with N-Boc-1,4-diaminobutane (3.9 g, 21 mmol) and stirred for 2 h under nitrogen. NaBH₃CN (3.9 g, 63 mmol) was then added in portions, and the reaction mixture was stirred at room temperature. After 3 d, the solution was neutralized with 1 M HCl solution to pH 7. The Boc-amine-terminated Dex- TA (denoted as Dex-TA-NH-Boc) was purified by ultrafiltration (MWCO 1000) and isolated as a white foam after freeze-drying. Yield: 4.4 g (88%). 1H NMR (D₂O): δ 1.3-14. (Boc,-C(CH3)), 1.4-1.7 (-NHCH₂-C₂H₄-CH₂-NH-), 2.6 and 3.0 (-C₂H₄-C₆H₄-OH and -NH-CH₂-C₂H₄-CH₂-NH-Boc), 3.2-4.1 (dextran glucosidic protons), 5.0 (dextran anomeric proton), 6.9 and 7.2 (-C₂H₄- C₆H₄-OH). In the second step, Dex-TA-NH-Boc (4.4 g) was dissolved in 110 mL of deionized water and after addition of 4.4 mL of TFA, the mixture was stirred overnight under nitrogen. The solution was then neutralized with 4 M NaOH to pH 7. The obtained amine-terminated Dex-TA (denoted as Dex-TA- NH₂) was purified by ultrafiltration (MWCO 1000) and subsequently freeze-dried. Yield: 3.4 g (78%). 1H NMR (D₂O): δ 1.5-1.6 (-NH- CH₂- C₂H₄- CH₂- NH₂), 2.6 and 3.0 (-C₂H₄- C₆H₄-OH and -NH- CH₂-C₂H₄- CH₂- NH₂), 3.2-4.1 (dextran glucosidic protons), 5.0 (dextran anomeric proton), 6.9 and 7.2 (-C₂H₄- C₆H₄-OH).

### Synthesis of hyaluronic acid grafted with Dex-TA

Copolymers of hyaluronic acid grafted with Dex-TA (denoted as HA-g-Dex-TA) were synthesized by a coupling reaction of Dex-TA-NH₂ with hyaluronic acid using EDAC/NHS as coupling reagent. Sodium hyaluronate (1 g) was dissolved in 50 mL of MES (0.1 M, pH 6.0), to which EDAC (1.8 g, 9.4 mmol) and NHS (1.1 g, 9.4 mmol) were added. After 30 min, a Dex-TA-NH₂ solution (1.25 g, in 10 mL of MES buffer) was added and the mixture was stirred under nitrogen for 3 d. The solution was then neutralized with 1 M NaOH to pH 7. To remove uncoupled Dex-TA-NH₂, the solution was ultrafiltrated (MWCO 10000), first with an aqueous solution of 50 mM NaCl and then deionized water. HA-g-Dex- TA was obtained as a white foam after freeze-drying. Yield: 1.9 g (84%). 1H NMR (D 2O): δ 1.5-1.6 (- NH-CH₂- C₂H₄-CH₂-NHCO-), 2.0 (-NHCO-CH3), 2.6 and 3.0 (-C₂H₄- C₆H₄-OH and -NH- CH₂- C₂H₄- CH₂-NHCO-), 3.2-4.1 (dextran and HA glucosidic protons), 4.4-4.6 (HA anomeric proton), 5.0 (dextran anomeric proton), 6.9 and 7.2 (-C₂H₄- C₆H₄-OH).

### Polymer characterization

1H NMR (300 MHz) spectra were recorded on a Varian Inova spectrometer (Varian, Palo Alto, USA). The signals of solvent residues were used as reference peaks for the 1H NMR chemical shift and were set at δ 4.79 for water. The degree of substitution (DS) of Dex-TA, which is defined as the number of tyramine moieties per 100 anhydroglucose rings in dextran, was determined using 1H NMR by comparing the integrals of signals at δ 5.0 (dextran anomeric proton) and δ 6.5-7.5 (tyramine aromatic protons). The number of grafted Dex-TA chains per HA molecule was determined using 1H NMR by comparing integrals of signals at δ 2.0 (acetamide methyl protons of HA) and δ 5.0 (dextran anomeric proton). The molecular weight and polydispersity of Dex-TA-NH₂, HA and HA-g-Dex-TA copolymers were determined by gel-permeation chromatography (GPC) relative to dextran standards (Fluka). GPC measurements were performed using a PL-GPC 120 Integrated GPC/SEC System (Polymer Labs) and two thermostated (30 °C) PL-aquagel-OH columns (8 µm, 300x7.5 mm, Polymer Labs). Sodium acetate buffer (NaAc, 300 mM, pH 4.5) containing 30% (v/v) methanol was used as eluent at a flow rate of 0.5 mL/min. 2.5

### Hydrogel formation and gelation time

Hydrogel samples (∼0.25 mL) were prepared in vials at 37 °C. In a typical example, to a PBS solution of HA-g-Dex-TA DS 10 (200 µL, 12.5 wt%), freshly prepared solutions of H₂O₂ (17.5 µL of 0.2 wt% stock solution) and HRP (32.5 µL of 11 unit/mL stock solution) in PBS were added and the mixture was gently vortexed. The final concentration of HA-g-Dex-TA was 10 wt%. In all experiments 0.25 mg HRP per mmol phenol groups and a H₂O₂/phenol molar ratio of 0.2 were applied. The time to form a gel (denoted as gelation time) was determined using the vial tilting method. No flow within 1 min upon inverting the vial was regarded as the gel state. The experiments were preformed in triplicate.

### Swelling and enzymatic degradation

For the swelling test, hydrogels (∼0.25 mL) of HA-g-Dex-TA were prepared as described above and freeze-dried (Wd). Subsequently, 2 mL of PBS solutions were applied to the dried hydrogels, which were then incubated at 37 °C for 72 h to reach the swelling equilibrium. The buffer solution was then removed from the samples and the hydrogels were weighed (Ws). The experiments were performed in triplicate and the degree of swelling was expressed as (Ws-Wd)/Wd. In degradation experiments, 2 mL of PBS containing 100 U/mL hyaluronidase was placed on top of 0.25 mL of the prepared hydrogels and the samples were then incubated at 37 °C. At regular time intervals, the buffer solution was removed from the samples and the hydrogels were weighed. The remaining gel (%) was calculated from the original gel weight after preparation (Wi) and remaining gel weight after exposure to the enzyme containing buffer (Wt), expressed as Wt/Wix100%. The buffer was replaced every 2-3 days and the experiments were performed in triplicate.

### Rheological analysis

Rheological experiments were carried out with a MCR 301 rheometer (Anton Paar) using parallel plates (25 mm diameter, 0°) configuration at 37°C in the oscillatory mode. In a typical example, 52.5 µL of a H₂O₂ stock solution and 97.5 µL of a HRP stock solution in PBS were mixed. The HRP/ H₂O₂ solution was then immediately mixed with 600 µL of a solution of HA-g-Dex-TA (12.5 wt%, in PBS) using a double syringe (2.5 mL, 1:4 volume ratio) equipped with a mixing chamber (Mixpac). After the samples were applied to the rheometer, the upper plate was immediately lowered to a measuring gap size of 0.5 mm, and the measurement was started. To prevent evaporation, a layer of oil was introduced around the polymer sample. A frequency of 0.5 Hz and a strain of 0.1% were applied in the analysis. The measurement was allowed to proceed until the storage moduli reached a plateau value.

### Chondrocyte isolation and incorporation

Bovine chondrocytes were isolated as previously reported [18] and cultured in chondrocyte expansion medium (DMEM with 10% heat inactivated fetal bovine serum, 1% penicillin/streptomycin (Gibco), 0.5 9 mg/mL fungizone (Gibco), 0.01 M MEM nonessential amino acids (Gibco), 10 mM HEPES and 0.04 mM L-proline) at 37 °C in a humidified atmosphere (95% air/5% CO₂). Hydrogels containing chondrocytes were prepared under sterile conditions by mixing a HA-g-Dex-TA /cell suspension with HRP/ H₂O₂. Solutions of HA-g-Dex-TA were made using medium and HRP and H₂O₂ stock solutions were made using PBS. All the components were sterilized by filtration through filters with a pore size of 0.22 µm. Chondrocytes (P1) were incorporated in the hydrogels using the same procedure as that in the absence of cells. The cell/gel constructs were prepared in vials. The final concentration of HA-g-Dex-TA was 10 wt% and the cell seeding density in the gels was 5×106/mL. After gelation, 1 mL of chondrocyte differentiation medium (DMEM with 0.1µM dexamethasone (Sigma), 100 µg/mL sodium pyruvate (Sigma), 0.2 mM ascorbic acid, 50 mg/mL insulin-transferrinselenite (ITS+1, Sigma), 100 U/ml penicillin, 100 µg/ml streptomycin, 10 ng/mL transforming growth factor ß3 (TGF-ß3, Invitrogen)) was added on top of the hydrogels and the constructs were incubated at 37°C in a humidified atmosphere containing 5% CO₂. The medium was replaced every 3 or 4 days.

### Cell viability and SEM

The effect of hydrogels on cell survival was studied using a Live-dead assay. At days 1, 7, 14 and 21, the hydrogel constructs were rinsed with PBS and stained with calcein AM/ethidium homodimer using the Live-dead assay Kit (Invitrogen), according to the manufactures' instructions. Hydrogel/cell constructs were visualized using fluorescence microscopy (Zeiss). As a result living cells fluoresce green and the nuclei of dead cells red. The morphology of the chondrocytes in the hydrogels was studied using a Philips XL 30 ESEM-FEG scanning electron microscope (SEM). After 21 days' in vitro culturing in differentiation medium, the hydrogel/cell constructs were fixed with formalin followed by sequential dehydration and critical point 10 drying. These samples were gold sputtered (Carringdon) and analyzed with SEM.

### Hydrogel degradation in the presence of chondrocytes

The gel/cell constructs (0.1 mL) were prepared in vials as described above and weighed (Wci). About 1 mL of chondrocyte differentiation medium was added on top of the gel and the constructs were incubated at 37 °C in a humidified atmosphere containing 5% CO₂. The medium was replaced every 3-4 days and the cell/gel constructs were weighed at regular time intervals (Wct). The swelling ratio of constructs was calculated from Wct/Wci. Afterwards, the constructs were washed extensively with water to remove the salts from the medium and then freeze-dried (Wcdt). The degradation profiles of the hydrogels with chondrocytes were based on the dry gel mass which was normalized to the original wet gel weight (Wci), expressed as Wcdt/Wci×100%. Dex-TA DS 15 hydrogels with chondrocytes were used as a control under the same conditions.

### Matrix production

After 1, 7, 14 and 21 days, samples were washed with PBS and frozen at -80 °C. After thawing, the constructs were digested with proteinase-K solution at 56 °C (>16 h). Quantification of total DNA was done by Cyquant dye kit (Molecular Probes) using a fluorescent plate reader (Perkin-Elmer). The amount of GAG was determined spectrophotometrically after reaction with dimethylmethylene blue dye (DMMB, Sigma-Aldrich). The intensity of the color was quantified immediately with a microplate reader (EL 312e Bio-TEK Instruments) by measuring the absorbance at 540 nm. The amount of GAG was calculated using a standard of chondroitin sulphate A or B (Sigma-Aldrich). The total collagen content was determined using the hydroxyproline assay in which hydroxyproline makes up 12.5% of collagen [32]. The hydroxyproline content was determined via a colorimetric assay by reaction with 11 chloramine T and dimethylaminobenzaldehyde. All values were corrected for the background staining of gels without cells and normalized to the dry gel mass (expressed as GAG or collagen (µg)/mg dry gel) or DNA content (expressed as GAG or collagen (µg)/DNA (µg)). Data (n=3, measured in triplicate) are expressed as mean ± standard deviation (SD).

### Biocompatibility of Dex-HA hydrogels

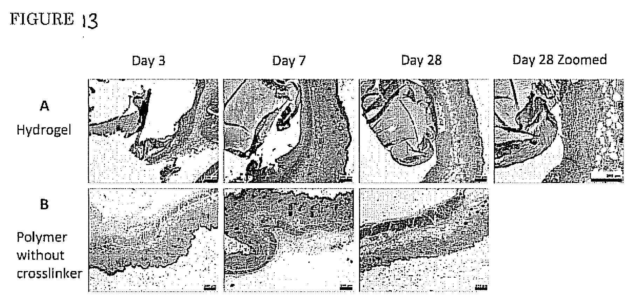

Biocompatibility of Dex-HA hydrogels was evaluated by subcutaneous implantation of in situ cross linked hydrogels in immuno-competent mice. Uncrosslinked polymer was also subcutaneously injected. The mice were sacrificed after 3, 7 and 28 days. Histological evaluation of representative samples, as shown in figure 13, indicates the formation of a thin fibrous capsule around the Dex-HA hydrogel as a result of the tissue response to the hydrogel. The diameter of this capsule decreases throughout time of implantation, with almost no eosinophils nor multinucleated cells being present in the hydrogel surroundings at day 28. This decrease in tissue response is indicative of a suitable material integration. There was no evidence for an acute reaction towards the hydrogel. These data strongly suggest that the hydrogel is biocompatible.

After verifying that Dex-HA is biocompatible, the chondrogenic potential of this hydrogel was evaluated. Dex-HA hydrogels were subcutaneous implanted in nude mice with and without chondrocytes. Samples were explanted after 28 days and representative sections of 10 µm were stained with Safranin O to determine the content in glycosaminoglycans. The strong red colour shown in figure 14 of Dex-HA with chondrocytes, shows that a high amount of glycosaminoglycans is being deposited by the cells. Thus, this hydrogel induces matrix deposition. The pore size of the hydrogels without cells is significantly higher than the ones observed when cells are present. This is due to the fact that all the pores are almost completely filled with de novo produced cartilaginous matrix. The neo-cartilage formation within the hydrogels with chondrocytes appears to be replacing the Dex-HA hydrogel, as this biomaterial slowly degrades.

Overall, Dex-HA hydrogels appear to be biocompatible and allow significant amount of cartilaginous matrix deposition *in vivo.*

### Statistical analysis

The experimental data between two groups were analyzed using a Student's t-test. Those among three or more groups were analyzed using One-way Analysis of Variance (ANOVA) with Turkey's post-hoc analysis. Statistical significance was set to a p value ≤ 0.05. Results are presented as mean ± standard deviation.

## Claims

1. A copolymer of hyaluronic acid (HA) grafted with a dextran-tyramine (Dex-TA) conjugate.

2. A copolymer according to claim 1, wherein said dextran-tyramine conjugate has a degree of substitution (DS), defined as the number of conjugated tyramine moieties per 100 anhydroglucose rings between 5 and 25.

3. A copolymer according to claim 1 or 2, wherein the dextran chain in said dextran-tyramine conjugate has an average molecular weight between 5 and 80 kDa.

4. A copolymer according to claim 1 for use as a medicament, preferably for the treatment of a subject in need of an implant.

5. A method for preparing a copolymer according to any of the preceding claims, comprising steps of
- modifying a dextran-tyramine conjugate at the reducing terminal glucose residue with an excess of N-Boc-1,4-diaminobutane;
- subjecting said modified dextran-tyramine conjugate to reductive amination using sodium cyanoborohydride;
- deprotecting the Boc group using trifluoroacetic acid to obtain a conjugate with a terminal free primary amine group (denoted as Dex-TA-NH₂).

6. A composition comprising a copolymer according to anyone of claims 1-4 with a suitable amount of hydrogen peroxide and suitable amount of a peroxidase, wherein the amount of peroxidase preferably is between 0.1 and 10 Units/ml, and wherein the concentration of hydrogen peroxide is preferably between 0.001 and 0.01 M.

7. A composition according to claim 6, wherein the amount of said copolymer is between 5 and 30 wt %.

8. A composition according to claim 6 or 7 in the form of an injectable hydrogel.

9. A composition according to anyone of claims 6-8 further comprising cells, preferably stem cells and or chondrocytes, wherein said stem cells are more preferably mesenchymal stem cells, embryonic stem cells or pluripotent stem cells or a combination of different stem cells and/or a growth factor, preferably a Bone Morphogenetic Protein (BMP) or a Transforming Growth Factor beta (TGF-β).

10. A composition according to anyone of claims 6-9 for use as a medicament, preferably for the treatment of a subject in need of an implant.

11. Method of producing a hydrogel, said method comprising the step of curing the composition according to any one of claims 6-10, wherein said composition comprises a curing amount of peroxide and a peroxidase.

12. Hydrogel obtainable by the method of claim 11.

## Patentansprüche

1. Kopolymer von Hyaluronsäure (HA), gepfropft mit einem Dextran-Tyramin (Dex-TA)-Konjugat.

2. Kopolymer nach Anspruch 1, wobei das Dextran-Tyramin-Konjugat einen Substitutionsgrad (DS), definiert als die Anzahl konjugierter Tyraminreste pro 100 Anhydroglukose-Ringen, zwischen 5 und 25 hat.

3. Kopolymer nach Anspruch 1 oder 2, wobei die Dextrankette in dem Dextran-Tyramin-Konjugat ein mittleres Molekulargewicht zwischen 5 und 80 kDa hat.

4. Kopolymer nach Anspruch 1 zur Verwendung als ein Arzneimittel, bevorzugt zur Behandlung eines Patienten, der ein Implantat benötigt.

5. Verfahren zur Herstellung eines Kopolymers nach einem der vorhergehenden Ansprüche, umfassend folgende Schritte:
- Modifizieren eines Dextran-Tyramin-Konjugats am reduzierenden endständigen Glukoserest mit einem Überschuss an N-Boc-1,4-diaminobutan;
- Unterziehen des modifizierten Dextran-Tyramin-Konjugats einer reduktiven Aminierung unter Verwendung von Natriumcyanborhydrid;
- Entschützen der Boc-Gruppe unter Verwendung von Trifluoressigsäure, um ein Konjugat mit einer endständigen freien primären Aminogruppe (bezeichnet als Dex-TA-NH₂) zu erhalten.

6. Zusammensetzung, umfassend ein Kopolymer nach einem der Ansprüche 1-4 mit einer geeigneten Menge von Wasserstoffperoxid und einer geeigneten Menge einer Peroxidase, wobei die Menge an Peroxidase bevorzugt zwischen 0,1 und 10 Einheiten/ml liegt und wobei die Konzentration von Wasserstoffperoxid bevorzugt zwischen 0,001 und 0,01 M liegt.

7. Zusammensetzung nach Anspruch 6, wobei die Menge des Kopolymers zwischen 5 und 30 Gew.-% liegt.

8. Zusammensetzung nach Anspruch 6 oder 7 in der Form eines injizierbaren Hydrogels.

9. Zusammensetzung nach einem der Ansprüche 6-8, ferner umfassend Zellen, bevorzugt Stammzellen und oder Chondrozyten, wobei die Stammzellen bevorzugter mesenchymale Stammzellen, embryonische Stammzellen oder pluripotente Stammzellen oder eine Kombination verschiedener Stammzellen sind, und/oder einen Wachstumsfaktor, bevorzugt ein knochenmorphogenetisches Protein (engl. Bone Morphogenetic Protein, BMP) oder einen transformierenden Wachstumsfaktor Beta (engl. Transforming Growth Factor beta, TGF-ß).

10. Zusammensetzung nach einem der Ansprüche 6-9 zur Verwendung als ein Arzneimittel, bevorzugt zur Behandlung eines Patienten, der ein Implantat benötigt.

11. Verfahren zur Herstellung eines Hydrogels, das Verfahren umfassend den Schritt, in dem die Zusammensetzung nach einem der Ansprüche 6-10 gehärtet wird, wobei die Zusammensetzung eine härtende Menge Peroxid und eine Peroxidase umfasst.

12. Hydrogel, erhältlich durch das Verfahren nach Anspruch 11.

## Revendications

1. Copolymère d'acide hyaluronique (AH) et de conjugué dextrane-tyramine (Dex-TA) greffé dessus.

2. Copolymère conforme à la revendication 1, dans lequel ledit conjugué dextrane-tyramine présente un degré de substitution (DS), défini comme étant le nombre de motifs de tyramine conjugués pour 100 cycles de type anhydroglucose, qui vaut entre 5 et 25.

3. Copolymère conforme à la revendication 1 ou 2, dans lequel la chaîne de dextrane, dans ledit conjugué dextrane-tyramine, présente une masse molaire moyenne qui vaut entre 5 et 80 kDa.

4. Copolymère conforme à la revendication 1 pour utilisation en tant que médicament, de préférence pour le traitement d'un sujet qui a besoin d'un implant.

5. Procédé de préparation d'un copolymère conforme à l'une des revendications précédentes, comportant les étapes suivantes :
- modifier un conjugué dextrane-tyramine, au niveau du résidu glucose terminal réducteur, avec du N-Boc-1,4-diamino-butane en excès ;
- soumettre ledit conjugué dextrane-tyramine modifié à une amination réductrice, réalisée au moyen de cyanoborohydrure de sodium ;
- éliminer le groupe protecteur Boc à l'aide d'acide trifluoroacétique, pour obtenir un conjugué porteur d'un groupe amino primaire libre terminal (dénommé Dex-TA-NH₂).

6. Composition comprenant un copolymère conforme à l'une des revendications 1 à 4, ainsi que du peroxyde d'hydrogène en une quantité appropriée et une peroxydase en une quantité appropriée, cette quantité de peroxydase valant de préférence entre 0,1 et 10 unités/mL et la concentration du peroxyde d'hydrogène valant de préférence entre 0,001 M et 0,01 M.

7. Composition conforme à la revendication 6, dans laquelle la proportion dudit copolymère vaut entre 5 et 30 % en poids.

8. Composition conforme à la revendication 6 ou 7, qui se présente sous la forme d'un hydrogel injectable.

9. Composition conforme à l'une des revendications 6 à 8, qui comprend en outre des cellules, de préférence des cellules souches et/ou des chondrocytes, lesquelles cellules souches sont, mieux encore, des cellules souches mésenchymateuses, des cellules souches embryonnaires ou des cellules souches pluripotentes ou une combinaison de différentes cellules souches, et/ou un facteur de croissance, de préférence une protéine BMP (protéine de morphogenèse osseuse) ou un facteur TGF-β) (facteur de croissance transformant bêta).

10. Composition conforme à l'une des revendications 6 à 9 pour utilisation en tant que médicament, de préférence pour le traitement d'un sujet qui a besoin d'un implant.

11. Procédé de préparation d'un hydrogel, lequel procédé comporte une étape de durcissement d'une composition conforme à l'une des revendications 6 à 10, laquelle composition comprend un peroxyde et une peroxydase en des quantités appropriées pour le durcissement.

12. Hydrogel accessible par un procédé conforme à la revendication 11.
